# EUROPÄISCHE PATENTSCHRIFT

(11) **EP 2 742 927 B1**
(45) Veröffentlichungstag und Bekanntmachung des Hinweises auf die Patenterteilung: **15.08.2018**
(21) Anmeldenummer: 13192618.0
(22) Anmeldetag: 13.11.2013
(51) Int. Cl.: A61K 8/34, A61K 8/37, A61K 8/55, A61K 8/92, A61K 8/97, A61K 8/06, A61Q 19/00

(54) **Emulsion mit Oligomeren einer Flavan-3-ol-Verbindung, einer speziellen Phenylcarbonyl-Tanninverbidnung und Ginsengextrakt**
Emulsion with oligomers of a flavan-3-ol- compound, a special phenylcarbonyl tannin compound and ginseng extract
Émulsion comprenant des oligomères d'une liaison flavan-3-ol, d'une liaison spéciale phénylcarbonyl-tanin et de l'extrait de ginseng

(30) Priorität: 17.12.2012 DE 102012223405
(43) Veröffentlichungstag der Anmeldung: 18.06.2014
(73) Patentinhaber: Henkel AG & Co. KGaA, 40589 Düsseldorf (DE)
(72) Erfinder: Bähren, Annika, 41363 Jüchen (DE); Stadler, Iris Marina, 47249 Duisburg (DE); Waldmann-Laue, Marianne, 40789 Monheim (DE)

(56) Entgegenhaltungen:
- EP-A2- 2 457 557
- US-A1- 2006 182 708

## Beschreibung

Die Erfindung betrifft emulsionsförmige Zusammensetzungen, die in Kosmetika, beispielsweise für die Haut oder die Haare, Verwendung finden.

Viele wasserhaltige Zusammensetzungen, insbesondere in Form von Kosmetika, umfassen Öl- oder Fettkomponenten. Diese Öl- oder Fettkomponenten sind nicht wasserlöslich und werden daher z.B. in wasserhaltigen Kosmetika meist mit Hilfe von Emulgatoren in Form einer Emulsion eingearbeitet. Öl- oder Fettkomponenten werden einerseits zur Pflege des Substrats, insbesondere der Haut oder Haare, eingesetzt. Andererseits lassen sich in Ölen oder Fetten öllösliche Wirkstoffe, wie z.B. UV-Filter, in ein wasserhaltiges Kosmetikum in gelöster Form einarbeiten. Die in der Öl- oder Fettkomponente gelösten Wirkstoffe können in dieser Form gleichmäßiger auf das Substrat aufgebracht werden. Auf diese Weise kann sich die Wirkung des Wirkstoffes am Substrat verbessert entfalten.

Die Druckschrift WO-A1-2002/089758 betrifft die Verwendung von oligomeren Procyanidinen in Hautpflegemitteln.

Die Druckschrift WO-A1-2010/139887 betrifft oligomere Procyanidine aus Pinien-Extrakt, welche durch Veresterung modifiziert wurden und gegen Hautalterung Einsatz finden.

Die Druckschrift WO-A-2005/011716 betrifft den Einsatz von prebiotisch wirksamen Pflanzenextrakten zur Förderung des Wachstums erwünschter Hautkeime.

Aus der EP 2 457 557 A2 ist die Verwendung von Kaliumcetylphosphat in Kombination mit hydrierten Palmölglyceriden zur Herstellung von Emulsionen bekannt.

Aufgabe der vorliegenden Erfindung ist es, wasserhaltige Emulsionen bereitzustellen, die stabil sind. Die Stabilität soll insbesondere in einem Temperaturintervall von 0°C bis 40°C, insbesondere von - 10,0 °C bis + 60°C vorliegen. Die Emulsion soll insbesondere über einen Zeitraum von mehreren Monaten, bevorzugt wenigstens 12 Wochen, stabil bleiben.

Es wurde überraschender Weise gefunden, dass eine Emulsion enthaltend besagte Wirkstoffkombination und eine spezielle Emulgatorkombination eine hervorragende Stabilität der Emulsion bewirkt. Ferner bleibt die erzielte Viskosität der Emulsion erhalten und ist stabil. Darüber hinaus soll die Emulsion einen hervorragenden Wirkungsgrad der Wirkstoffkombination auf dem Substrat bewirken.

Ein erster Gegenstand der Erfindung ist eine kosmetische Zusammensetzung in Form einer Emulsion, enthaltend
a) mindestens ein Oligomeres einer Flavan-3-ol-Verbindung
   und
b) mindestens eine Verbindung umfassend mindestens ein Strukturfragment der Formel (T1) 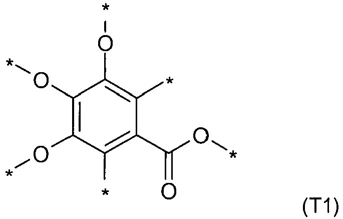 worin eine mit einem * gekennzeichnete Bindung für eine freie Valenz des Strukturfragments der Verbindung steht
   und
c) Ginsengextrakt
   und
d) mindestens ein Salz von C₁₂₋₂₀-Alkylphosphat, insbesondere ein Salz von Cetylphosphat,
   und
e) mindestens ein C₁₄₋₂₀-Mono- oder Diacylglycerid, bevorzugt mindestens ein C₁₆₋₁₈-Mono- oder Diacylglycerid, besonders bevorzugt ausgewählt aus gehärteten Palmölglyceriden,
   und
f) Wasser,
wobei die Salze von C₁₂₋₂₀-Alkylphosphat und die C₁₄₋₂₀-Mono- und C₁₄₋₂₀-Diacylglyceride in einem Gewichtsverhältnis C₁₂₋₂₀-Alkylphosphat-Salz zu besagtem Glycerid von 3 zu 1 bis 1 zu 1, enthalten sind.

Es ist erfindungsgemäß bevorzugt, wenn Wasser in der Emulsion in der kontinuierlichen Phase enthalten ist.

Bevorzugt liegen die erfindungsgemäßen kosmetischen Zusammensetzungen in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion vor. Erfindungsgemäß besonders bevorzugte kosmetische Zusammensetzungen liegen in Form einer Öl-in-Wasser Emulsion vor.

Weiterhin ist es bevorzugt, wenn die kosmetische Zusammensetzung mindestens 20 Gew.-%, besonders bevorzugt mindestens 40 Gew.-%, ganz besonders bevorzugt mindestens 50 Gew.-% Wasser, insbesondere jeweils in der kontinuierlichen Phase, enthält.

Die erfindungsgemäße Zusammensetzung enthält zwingend mindestens ein Oligomeres einer Flavan-3-ol-Verbindung.

Unter Oligomeren sind erfindungsgemäß Verbindungen zu verstehen, die aus 2 bis 15 sich wiederholenden Monomeren Bausteinen bestehen.

Unter Flavan-3-ol-Verbindungen fallen erfindungsgemäß sowohl die Verbindungen der Catechine als auch Verbindungen der Leukoanthocyanidine (bevorzugt Delphinidin).

Bevorzugte oligomere Flavan-3-ol-Verbindungen werden ausgewählt aus mindestens einem oligomeren Proanthocyanidin. Die Bezeichnung Proanthocyanidin leitet sich von deren Eigenschaften ab. Proanthocyanidine ergeben bei Erhitzung mit Säuren und in Anwesenheit von Sauerstoff die entsprechenden Anthocvanidine.

Bevorzugt sind im Rahmen der vorliegenden Erfindung Dimere von Flavan-3-ol-Verbindungen und/oder Trimere von Flavan-3-ol-Verbindungen und/oder Tetramere von Flavan-3-ol-Verbindungen (bevorzugt Dimere von Delphinidin und/oder Trimere von Delphinidin und/oder Tetramere von Delphinidin).

Als besonders bevorzugte oligomere Proanthocyanidine gelten diejenigen, die aus den Blättern von *Ribes Nigrum* extrahiert werden. Daher enthält die erfindungsgemäße Zusammensetzung besonders bevorzugt einen (insbesondere mit Wasser und/oder Ethanol als Extraktionsmittel gewonnenen) Extrakt der Blätter von *Ribes Nigrum,* enthaltend mindestens ein oligomeres Proanthocyanidin.

Die erfindungsgemäßen Zusammensetzungen enthalten die Oligomere einer Flavan-3-ol-Verbindung bezogen auf die gesamte Zusammensetzung bevorzugt in einer Gesamtmenge von 0,00005 Gew.-% bis 0,1 Gew.-%, insbesondere bevorzugt von 0,0001 Gew.-% bis 0,05 Gew.-%.

Die erfindungsgemäßen Zusammensetzungen enthalten besonders bevorzugt einen (insbesondere mit Wasser und/oder Ethanol als Extraktionsmittel gewonnenen) Extrakt der Blätter von *Ribes Nigrum* bezogen auf die gesamte Zusammensetzung bevorzugt in einer Gesamtmenge von 0,001 Gew.-% bis 0,2 Gew.-%, insbesondere bevorzugt von 0,005 Gew.-% bis 0,02 Gew.-%.

Weiterhin enthält die erfindungsgemäße Zusammensetzung zwingend mindestens eine Verbindung umfassend mindestens ein Strukturelement der Formel (T1) 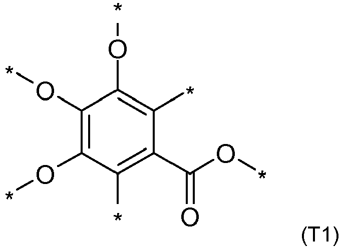 worin eine mit einem * gekennzeichnete Bindung für eine freie Valenz des Strukturfragments der Verbindung steht.

Bevorzugte Verbindungen der Formel (T1) werden ausgewählt aus mindestens einer Verbindung der Gruppe, die gebildet wird aus Gallussäure, Ellagsäure, Gallotanninen und Ellagitanninen.

Besonders bevorzugt enthält die erfindungsgemäße Zusammensetzung mindestens eine Verbindung der Formel (T1) aus einem Extrakt der Rinde von *Pinus Sylvestris.* Daher enthält die erfindungsgemäße Zusammensetzung besonders bevorzugt einen (insbesondere mit Wasser und/oder Ethanol als Extraktionsmittel gewonnenen) Extrakt der Rinde von *Pinus Sylvestris,* enthaltend mindestens eine Verbindung der obigen Formel (T1).

Die erfindungsgemäßen Zusammensetzungen enthalten Verbindungen der Formel (T1) bezogen auf die gesamte Zusammensetzung bevorzugt in einer Gesamtmenge von 0,00005 Gew.-% bis 0,1 Gew.-%, insbesondere bevorzugt von 0,0001 Gew.-% bis 0,05 Gew.-%.

Die erfindungsgemäßen Zusammensetzungen enthalten besonders bevorzugt einen (insbesondere mit Wasser und/oder Ethanol als Extraktionsmittel gewonnenen) Extrakt der Rinde von *Pinus Sylvestris* bezogen auf die gesamte Zusammensetzung bevorzugt in einer Gesamtmenge von 0,001 Gew.-% bis 0,2 Gew.-%, insbesondere bevorzugt von 0,005 Gew.-% bis 0,02 Gew.-%.

Als weitere zwingende Komponente enthält die erfindungsgemäße Zusammensetzung Ginsengextrakt.

Unter Ginseng wird erfindungsgemäß die Wurzel einer Pflanze aus der Familie der Araliengewächse der Gattung Ginseng verstanden. Zu diesen Pflanzen gehört unter anderem der *Araliacee Panax ginseng* C. A. Meyer, der sogenannte echte Ginseng, sowie *Panax japonicus* und *Panax Pseudoginseng.*

Es ist erfindungsgemäß bevorzugt, dass der Ginsengextrakt mindestens ein Sapogenin enthält. Dabei handelt es sich bevorzugt um Sapogenine, die ausgewählt sind aus mindestens einer Verbindung gemäß Formel (I) und/oder mindestens einem glykosidischen Derivat davon 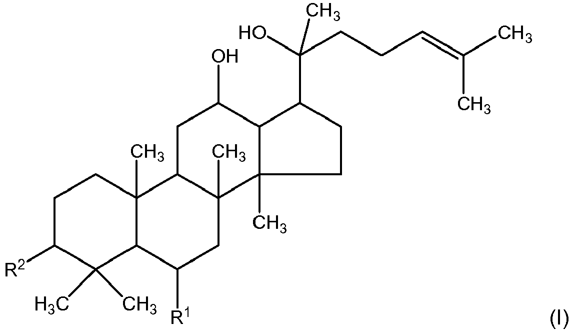 worin
R¹ und R² unabhängig voneinander ein Wasserstoffatom oder eine Hydroxygruppe bedeuten.

Wenn die Verbindung der Formel (I) als glycosidisches Derivat vorliegt, dann bindet die Kohlenhydrateinheit an eine der Hydroxygruppen des Moleküls mit der Formel (I). Die glykosidischen Derivate der Verbindungen der Formel (I) leiten sich bevorzugt ab von Glucopyranose, Arabinopyranose, Arabinofuranose, Xylofuranose, Xylopyranose und/oder Rhamnopyranose. Dabei sind jeweils L- oder D-Stereomere möglich. Besonders bevorzugt leiten sich die glykosidischen Derivate der Verbindungen mit der Formel (I) ab von D-Glucopyranose, L-Arabinopyranose, L-Arabinofuranose, D-Xylofuranose, D-Xylopyranose und/oder L-Rhamnopyranose.

Besonders bevorzugte Verbindungen mit der Formel (I) oder deren glykosidischen Derivate sind Betulafolientriol, Protopanaxadiol und/oder Protopanaxatriol.

Als verwendbare Extrakte des Ginsengs sind beispielsweise die Handelsprodukte Extrapon® Ginseng (INCI-Bezeichnung: Aqua (Water), Ethoxydiglycol, Propylene Glycol, Butylene Glycol, Panax Ginseng Root Extract, Lactic Acid, Glucose) der Firma Symrise oder Novaplant® Ginseng (INCI-Bezeichnung: Aqua (Water), Butylene Glycol, Panax Ginseng Root Extract) der Firma Crodarom zu nennen.

Ein besonders bevorzugter Ginsengextrakt wird von der Firma Cosmetochem unter dem Handelsnamen Herbasol® Extrakt Ginseng (INCI-Bezeichnung: Isopropylmyristate, Panax Ginseng Root Extract) als öllöslicher Ginsengextrakt vertrieben.

Bezogen auf das Gewicht der gesamten Zusammensetzung ist der Ginsengextrakt bevorzugt in einer Menge von 0,25 Gew.-% bis 1,00 Gew.-%, insbesondere 0,3 Gew.-% bis 0,6 Gew.-%, in der erfindungsgemäßen Zusammensetzung enthalten.

Die erfindungsgemäße kosmetische Zusammensetzung enthält zwingend mindestens ein Salz von C₁₂₋₂₀-Alkylphosphaten, insbesondere mindestens ein Salz des Cetylphosphats.

Salze von C₁₂₋₂₀-Alkylphosphaten, umfassen Phosphorsäure-C₁₂₋₂₀-Monoalkylester und Phosphorsäure-C₁₂₋₂₀-Dialkylester.

Die Salze von C₁₂₋₂₀-Alkylphosphaten können beispielsweise als Alkali-, Erdalkali-, Ammonium-, Alkylammonium-, Alkanolamin- oder Glucammoniumsalz vorliegen. Bevorzugt sind die entsprechenden Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze, sowie Salze der basischen Aminosäuren Ornithin, Lysin, Arginin und/oder Histidin. Besonders bevorzugt sind die Kaliumsalze besagter C₁₂₋₂₀-Alkylphosphate.

Erfindungsgemäß bevorzugte kosmetische Zusammensetzungen sind dadurch gekennzeichnet, dass die Salze von C₁₂₋₂₀-Alkylphosphat, insbesondere die Salze von Cetylphosphat" in einer Gesamtmenge von 0,2 bis 2,0 Gew.-%, bevorzugt 0,3 bis 1,8 Gew.-%, besonders bevorzugt 0,3 bis 0,7 Gew.-%, jeweils bezogen auf das Gesamtgewicht der Zusammensetzung, enthalten sind.

Erfindungsgemäß bevorzugte Salze von C₁₂₋₂₀-Alkylphosphaten, sind ausgewählt aus den Monoestern von Phosphorsäure mit Laurylalkohol, Tridecylalkohol, Isotridecylalkohol, Myristylalkohol, Pentadecylalkohol, Cetylalkohol, Palmitylalkohol, Isocetylalkohol, Isostearylalkohol, Stearylalkohol, Oleylalkohol, Elaidylalkohol, Linoleylalkohol, Linolenylalkohol, Nonadecylalkohol, Arachylalkohol, Gadoleylalkohol oder Arachidonalkohol, die als Alkali-, Erdalkali-, Ammonium-, Alkylammonium-, Alkanolamin- oder Glucammoniumsalz, bevorzugt die entsprechenden Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze sowie als Salze der basischen Aminosäuren Ornithin, Lysin, Arginin und/oder Histidin, vorliegen. Bevorzugt sind die Kaliumsalze der genannten Phosphorsäuremonoester. Besonders bevorzugt ist Dikaliummonocetylphosphat.
Weitere erfindungsgemäß bevorzugte Salze von C₁₂₋₂₀-Alkylphosphaten sind ausgewählt aus den Diestern von Phosphorsäure mit Laurylalkohol, Tridecylalkohol, Isotridecylalkohol, Myristylalkohol, Pentadecylalkohol, Cetylalkohol, Palmitylalkohol, Isocetylalkohol, Isostearylalkohol, Stearylalkohol, Oleylalkohol, Elaidylalkohol, Linoleylalkohol, Linolenylalkohol, Nonadecylalkohol, Arachylalkohol, Gadoleylalkohol oder Arachidonalkohol, die als Alkali-, Erdalkali-, Ammonium-, Alkylammonium-, Alkanolamin- oder Glucammoniumsalz, bevorzugt die entsprechenden Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze sowie als Salze der basischen Aminosäuren Ornithin, Lysin, Arginin und/oder Histidin, vorliegen. Bevorzugt sind die Kaliumsalze der genannten Phosphorsäurediester. Besonders bevorzugt ist Kaliumdicetylphosphat.

Besonders bevorzugt sind Mischungen aus Mono-C₁₂₋₂₀-Alkylphosphaten und Di-C₁₂₋₂₀-Alkylphos-phaten. Außerordentlich bevorzugt sind Mischungen aus Dikaliummonocetylphosphat und Kaliumdicetylphosphat.

Die erfindungsgemäße kosmetische Zusammensetzung enthält zwingend mindestens ein C₁₄₋₂₀-Mono- oder Diacylglycerid, bevorzugt mindestens ein C₁₆₋₁₈-Mono- oder Diacylglycerid. Diese werden jeweils besonders bevorzugt aus gehärteten Palmölglyceriden ausgewählt. Sie tragen die INCI-Deklaration Hydrogenated Palm Glycerides.

Erfindungsgemäß bevorzugte C₁₄₋₂₀-Mono- oder Diacylglyceride sind ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid, Diarachinoylglycerid sowie Mischungen zweier oder meherer Verbindungen daraus. Weitere erfindungsgemäß bevorzugte C₁₄₋₂₀-Mono- oder Diacylglyceride sind Glyceride gehärteter, das heißt, hydrierter, bevorzugt vollständig hydrierter, Fettsäuren natürlicher Öle. Erfindungsgemäß besonders bevorzugt sind gehärtete Palmölglyceride.

Erfindungsgemäß bevorzugte kosmetische Zusammensetzungen enthalten die C₁₄₋₂₀-Mono- oder Diacylglyceride, bevorzugt die C₁₆₋₁₈-Mono- oder Diacylglyceride, besonders bevorzugt die gehärteten Palmölglyceride, in einer Gesamtmenge von 0,1 bis 1,7 Gew.-% bezogen auf das Gesamtgewicht der Zusammensetzung.

Die Salze von C₁₂₋₂₀-Alkylphosphat (insbesondere die Salze von Cetylphosphat) und die C₁₄₋₂₀-Mono- und C₁₄₋₂₀-Diacylglyceride sind in einem Gewichtsverhältnis C₁₂₋₂₀-Alkylphosphat-Salz zu besagtem Glycerid von 3 zu 1 bis 1 zu 1, bevorzugt von 2,5 zu 1 bis 1 zu 1, in der erfindungsgemäßen Zusammensetzung enthalten.

Es ist erfindungsgemäß besonders bevorzugt, wenn die erfindungsgemäße kosmetische Zusammensetzung
- die Salze von C₁₂₋₂₀-Alkylphosphat (insbesondere die Salze von Cetylphosphat) und die C₁₄₋₂₀-Mono- und C₁₄₋₂₀-Diacylglyceride in einem Bereich des Gewichtsverhältnisses C₁₂₋₂₀-Alkylphosphat-Salz zu besagtem Glycerid von 3 zu 1 bis 1 zu 1-, bevorzugt von 2,5 zu 1 bis 1 zu 1, und
- die Salze von C₁₂₋₂₀-Alkylphosphat, insbesondere die Salze von Cetylphosphat in einer Gesamtmenge von 0,2 bis 2,0 Gew.-%, bevorzugt 0,3 bis 1,8 Gew.-%, besonders bevorzugt 0,3 bis 0,7 Gew.-%, jeweils bezogen auf das Gesamtgewicht der Zusammensetzung, enthalten, und
- die C₁₆₋₁₈-Mono- oder Diacylglyceride, besonders bevorzugt die gehärteten Palmölglyceride, in einer Gesamtmenge von 0,1 bis 1,7 Gew.-% bezogen auf das Gesamtgewicht der Zusammensetzung,
enthält.

Zur stabilen Verdickung der erfindungsgemäßen kosmetischen Zusammensetzungen eignet sich besonders bevorzugt mindestens ein Verdickungsmittel, ausgewählt aus mindestens einem Polymer, welches durch Polymerisation von zumindest 2-Methyl-2[(1-oxo-2-propenyl)amino]-1-propansulfonsäure (AMPS) erhalten wird.
Die erfindungsgemäße kosmetische Zusammensetzung enthält besagte Polymere bevorzugt in einer Gesamtmenge von 0,005 bis 5,0 Gew.-%, insbesondere 0,005 - 2,5 Gew.-%, besonders bevorzugt 0,1 bis 2 Gew.-%, jeweils bezogen auf das Gewicht des Mittels.

Die erfindungsgemäßen kosmetischen Mittel dieser Ausführungsform enthalten bevorzugt zusätzlich mindestens ein Homopolymer oder Copolymer der 2-Methyl-2[(1-oxo-2-propenyl)amino]-1-propansulfonsäure (AMPS), dessen Säuregruppen teilweise oder vollständig neutralisiert sind. Besagtes Polymer ist wiederum bevorzugt vernetzt.

Aus der partiellen Neutralisation der Säuregruppen resultieren der neutralisierte Monomerbaustein, beispielsweise Natrium-2-methyl-2[(1-oxo-2-propenyl)amino]-1-propansulfonat oder Ammonium-2-methyl-2[(1-oxo-2-propenyl)amino]-1-propansulfonat, und der nicht-neutralisierte, saure Monomerbaustein, 2-Methyl-2[(1-oxo-2-propenyl)amino]-1-propansulfonsäure. Als neutralisiertes Monomer geeignet sind alle 2-Methyl-2[(1-oxo-2-propenyl)amino]-1-propansulfonatsalze. Bevorzugt sind das Natrium-, Kalium-, Ammonium-, Ethanolamin- oder Aminosäure-Salz. Besonders bevorzugt sind Natrium-2-methyl-2[(1-oxo-2-propenyl)amino]-1-propansulfonat und Ammonium-2-methyl-2[(1-oxo-2-propenyl)amino]-1-propansulfonat.

Ein erstes bevorzugtes Verdickungsmittel ist das vernetzte AMPS-Homopolymer Ammonium Polyacryloyldimethyl Taurate. Ein derartiges vernetztes AMPS-Homopolymer ist beispielsweise von Clariant unter der Bezeichnung Hostacerin AMPS erhältlich.

Besonders bevorzugt sind neben den vernetzten AMPS-Homopolymeren die vernetzten AMPS-Copolymere. Bevorzugte vernetzte AMPS-Copolymere enthalten neben AMPS ein, zwei oder drei, verschiedene Monomere, die neutral sein oder eine schwache Säurefunktion aufweisen können. Selbstverständlich sind auch vernetzte AMPS-Copolymere geeignet, die neben AMPS vier oder sogar mehr Monomere aufweisen.

In einer weiteren bevorzugten Ausführungsform der vorliegenden Erfindung enthält das verdikkende vernetzte AMPS-Copolymer als zweites Monomer ein neutrales Monomer, das ausgewählt ist aus 2-Hydroxyethylacrylat, 2,3-Dihydroxypropylacrylat, 2-Hydroxyethylmethacrylat, 2,3-Dihydroxypropylmethacrylat, Acrylamid, Alkylacrylamiden, wie beispielsweise Methylacrylamid, Ethylacrylamid, n-Propylacrylamid und Isopropylacrylamid, Dialkylamiden, wie beispielsweise Dimethylacrylamid, Diethylacrylamid, Di-n-propylacrylamid und Diisopropylacrylamid, den ethoxylierten Derivaten der vorstehend genannten Ester und Amide, sowie N-Vinylpyrrolidon. Besonders bevorzugt ist das neutrale Polyelektrolytmonomer ausgewählt aus 2-Hydroxyethylacrylat, 2-Hydroxyethylmethacrylat, 2,3-Dihydroxypropylmethacrylat, den ethoxylierten Derivaten der genannten Ester, Acrylamid, Dimethylacrylamid, sowie N-Vinylpyrrolidon. Außerordentlich bevorzugte neutrale Polyelektrolytmonomere sind ausgewählt aus 2-Hydroxyethylacrylat, Acrylamid, Dimethylacrylamid und Vinylpyrrolidon. Außerordentlich bevorzugte vernetzte AMPS-Copolymere sind ausgewählt aus vernetzten Copolymeren, bestehend aus AMPS und N-Vinylpyrrolidon, AMPS und 2-Hydroxyethylacrylat, AMPS und Acrylamid, AMPS und Dimethylacrylamid.

Besonders bevorzugte ethoxylierte Derivate der vorstehend genannten (Meth)Acrylsäureester und (Meth)Acrylsäureamide sind solche der nachstehenden Formel (AMPS-Copol), in denen R1 und R3, die gleich oder verschieden sind, ein Wasserstoffatom oder eine in etwa lineare oder verzweigte Alkylgruppe mit 1 bis 6 Kohlenstoffatomen bedeuten; Y O oder NH bedeutet; R2 eine Kohlenwasserstoffgruppe mit 6 bis 50 Kohlenstoffatomen ist; und x die Molzahl des Alkylenoxids angibt und im Bereich von 1 bis 50, bevorzugt 8 bis 20, liegt:

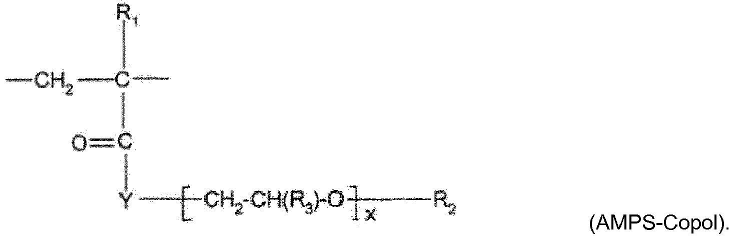

Ein bevorzugtes vernetztes Ammonium Acryloyldimethyltaurate/VP Copolymer ist von Clariant unter der Bezeichnung Aristoflex AVC erhältlich.

In einer weiteren ganz besonders bevorzugten Ausführungsform der vorliegenden Erfindung enthält das verdickende vernetzte AMPS-Copolymer als zweites Monomer ein Monomer mit schwacher Säurefunktion, das ausgewählt ist aus Acrylsäure, Methacrylsäure, Itaconsäure und Maleinsäure, wobei die schwache Säurefunktion teilweise oder vollständig neutralisiert ist und als Salz, bevorzugt als Natrium-, Kalium-, Ammonium-, Ethanolamin- oder Aminosäure-Salz, besonders bevorzugt als Natriumsalz, vorliegt. Weitere außerordentlich bevorzugte vernetzte AMPS-Copolymere sind ausgewählt aus vernetzten Copolymeren, bestehend aus AMPS und Natriumacrylat, AMPS und Natriummethacrylat, AMPS und Acrylamid und Natriumacrylat, AMPS und Acrylamid und Natriumacrylat und Acrylsäure.

Ein bevorzugtes vernetztes Sodium Acrylate/Sodium Acryloyldimethyl Taurate Copolymer ist von Seppic unter der Bezeichnung Simulgel EPG erhältlich.
Weitere bevorzugte vernetzte Copolymere mit 2-Methyl-2[(1-oxo-2-propenyl)amino]-1-propansulfonsäure (AMPS) sind kommerziell erhältlich, z. B. unter den Handelsnamen Simulgel®NS, Simulgel®I-NS 100, Simulgel®FL, Sepiplus®S, Simulgel®600, Sepiplus 400, Simulgel®SMS 88, Simulgel®EG und Simulgel®EPG von der Firma Seppic.

Folgende Ausführungsformen (A) bis (D) sind besonders bevorzugte Ausführungsformen der Erfindung:
(A): Kosmetische Zusammensetzung in Form einer Emulsion, enthaltend
   a) mindestens ein Oligomeres einer Flavan-3-ol-Verbindung
      und
   b) mindestens eine Verbindung umfassend mindestens ein Strukturfragment der Formel (T1) 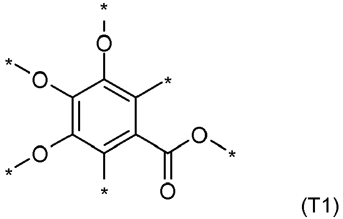 worin eine mit einem * gekennzeichnete Bindung für eine freie Valenz des Strukturfragments der Verbindung steht
      und
   c) Ginsengextrakt
      und
   d) 0,2 bis 2,0 Gew.-%, bevorzugt 0,3 bis 1,8 Gew.-%, besonders bevorzugt 0,3 bis 0,7 Gew.-%, mindestens eines Salzes von C₁₂₋₂₀-Alkylphosphat, insbesondere eines Salzes von Cetylphosphat,
      und
   e) 0,1 bis 1,7 Gew.-% mindestens eines C₁₄₋₂₀-Mono- oder Diacylglycerids, bevorzugt mindestens eines C₁₆₋₁₈-Mono- oder Diacylglycerids, besonders bevorzugt ausgewählt aus gehärteten Palmölglyceriden,
      und
   f) Wasser,
   und die Salze von C₁₂₋₂₀-Alkylphosphat (insbesondere die Salze von Cetylphosphat) und die C₁₄₋₂₀-Mono- und C₁₄₋₂₀-Diacylglyceride in einem Bereich des Gewichtsverhältnisses C₁₂₋₂₀-Alkylphosphat-Salz zu besagtem Glycerid von 3 zu 1 bis 1 zu 1, bevorzugt von 2,5 zu 1 bis 1 zu 1, enthalten sind.
(B): Kosmetische Zusammensetzung in Form einer Emulsion, enthaltend bezogen auf die gesamte Zusammensetzung jeweils in einer Gesamtmenge von
   a) 0,00005 Gew.-% bis 0,1 Gew.-%, insbesondere bevorzugt 0,0001 Gew.-% bis 0,05 Gew.-%, mindestens ein Oligomeres einer Flavan-3-ol-Verbindung
      und
   b) 0,00005 Gew.-% bis 0,1 Gew.-%, bevorzugt 0,0001 Gew.-% bis 0,05 Gew.-%, mindestens eine Verbindung umfassend mindestens ein Strukturfragment der Formel (T1) 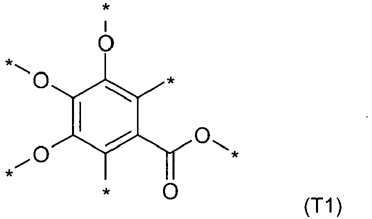 worin eine mit einem * gekennzeichnete Bindung für eine freie Valenz des Strukturfragments der Verbindung steht
      und
   c) Ginsengextrakt
      und
   d) 0,2 bis 2,0 Gew.-%, bevorzugt 0,3 bis 1,8 Gew.-%, besonders bevorzugt 0,3 bis 0,7 Gew.-%, mindestens eines Salzes von C₁₂₋₂₀-Alkylphosphat, insbesondere eines Salzes von Cetylphosphat,
      und
   e) 0,1 bis 1,7 Gew.-% mindestens eines C₁₄₋₂₀-Mono- oder Diacylglycerids, bevorzugt mindestens eines C₁₆₋₁₈-Mono- oder Diacylglycerids, besonders bevorzugt ausgewählt aus gehärteten Palmölglyceriden,
      und
   f) Wasser,
   und die Salze von C₁₂₋₂₀-Alkylphosphat (insbesondere die Salze von Cetylphosphat) und die C₁₄₋₂₀-Mono- und C₁₄₋₂₀-Diacylglyceride in einem Bereich des Gewichtsverhältnisses C₁₂₋₂₀-Alkylphosphat-Salz zu besagtem Glycerid von 3 zu 1 bis 1 zu 1, bevorzugt von 2,5 zu 1 bis 1 zu 1, enthalten sind.
(C): Kosmetische Zusammensetzung in Form einer Emulsion, enthaltend
   a) mindestens ein Oligomeres einer Flavan-3-ol-Verbindung
      und
   b) mindestens eine Verbindung umfassend mindestens ein Strukturfragment der Formel (T1) 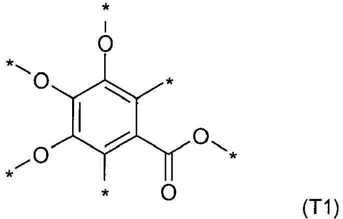 worin eine mit einem * gekennzeichnete Bindung für eine freie Valenz des Strukturfragments der Verbindung steht
      und
   c) Ginsengextrakt
      und
   d) 0,2 bis 2,0 Gew.-%, bevorzugt 0,3 bis 1,8 Gew.-%, besonders bevorzugt 0,3 bis 0,7 Gew.-%, mindestens eines Salzes von C₁₂₋₂₀-Alkylphosphat, insbesondere eines Salzes von Cetylphosphat,
      und
   e) 0,1 bis 1,7 Gew.-% mindestens eines C₁₄₋₂₀-Mono- oder Diacylglycerids, bevorzugt mindestens eines C₁₆₋₁₈-Mono- oder Diacylglycerids, besonders bevorzugt ausgewählt aus gehärteten Palmölglyceriden,
      und
   f) Wasser,
      und
   g) mindestens eines Verdickungsmittels, ausgewählt aus mindestens einem Polymer, welches durch Polymerisation von zumindest 2-Methyl-2[(1-oxo-2-propenyl)amino]-1-propansulfonsäure (AMPS) erhalten wird,
   und die Salze von C₁₂₋₂₀-Alkylphosphat (insbesondere die Salze von Cetylphosphat) und die C₁₄₋₂₀-Mono- und C₁₄₋₂₀-Diacylglyceride in einem Bereich des Gewichtsverhältnisses C₁₂₋₂₀-Alkylphosphat-Salz zu besagtem Glycerid von 3 zu 1 bis 1 zu 1, bevorzugt von 2,5 zu 1 bis 1 zu 1, enthalten sind.
(D): Kosmetische Zusammensetzung in Form einer Emulsion, enthaltend bezogen auf die gesamte Zusammensetzung jeweils in einer Gesamtmenge von
   a) 0,00005 Gew.-% bis 0,1 Gew.-%, insbesondere bevorzugt 0,0001 Gew.-% bis 0,05 Gew.-%, mindestens ein Oligomeres einer Flavan-3-ol-Verbindung
      und
   b) 0,00005 Gew.-% bis 0,1 Gew.-%, bevorzugt 0,0001 Gew.-% bis 0,05 Gew.-%, mindestens eine Verbindung umfassend mindestens ein Strukturfragment der Formel (T1) 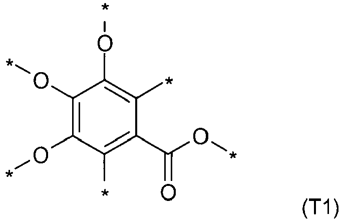 worin eine mit einem * gekennzeichnete Bindung für eine freie Valenz des Strukturfragments der Verbindung steht
      und
   c) Ginsengextrakt
      und
   d) 0,2 bis 2,0 Gew.-%, bevorzugt 0,3 bis 1,8 Gew.-%, besonders bevorzugt 0,3 bis 0,7 Gew.-%, mindestens eines Salzes von C₁₂₋₂₀-Alkylphosphat, insbesondere eines Salzes von Cetylphosphat,
      und
   e) 0,1 bis 1,7 Gew.-% mindestens eines C₁₄₋₂₀-Mono- oder Diacylglycerids, bevorzugt mindestens eines C₁₆₋₁₈-Mono- oder Diacylglycerids, besonders bevorzugt ausgewählt aus gehärteten Palmölglyceriden,
      und
   f) Wasser,
      und
   g) 0,005 bis 5,0 Gew.-%, insbesondere 0,005 - 2,5 Gew.-%, besonders bevorzugt 0,1 bis 2 Gew.-%, mindestens eines Verdickungsmittels, ausgewählt aus mindestens einem Polymer, welches durch Polymerisation von zumindest 2-Methyl-2[(1-oxo-2-propenyl)amino]-1-propansulfonsäure (AMPS) erhalten wird,
   und die Salze von C₁₂₋₂₀-Alkylphosphat (insbesondere die Salze von Cetylphosphat) und die C₁₄₋₂₀-Mono- und C₁₄₋₂₀-Diacylglyceride in einem Bereich des Gewichtsverhältnisses C₁₂₋₂₀-Alkylphosphat-Salz zu besagtem Glycerid von 3 zu 1 bis 1 zu 1, bevorzugt von 2,5 zu 1 bis 1 zu 1, enthalten sind.

Unabhängig von vorgenannten Ausführungsformen (A) bis (D) aber auch diese umfassend sind weitere erfindungsgemäß bevorzugte Zusammensetzungen dadurch gekennzeichnet, dass sie zusätzlich mindestens einen konditionierenden Wirkstoff enthalten. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen.

Die erfindungsgemäßen Zusammensetzungen enthalten bevorzugt mindestens einen Fettkörper. Als Fettkörper besonders bevorzugt mindestens einen Fettstoff. Unter sind Fettsäuren, Fettalkohole, natürliche und synthetische Wachse und natürliche und synthetische kosmetische Ölkomponenten zu verstehen.

Die Fettstoffe sind in den erfindungsgemäßen Zusammensetzungen bevorzugt in einer Menge von 5,0 bis 50,0 Gew.-%, besonders bevorzugt von 8,0 bis 30,0 Gew.-%, jeweils bezogen auf das Gesamtgewicht des Zusammensetzung, enthalten.

Als Fettstoff eignen sich bevorzugt lineare, gesättigte primäre Alkohole mit 14 - 30 Kohlenstoffatomen, Fettsäuren mit 6 bis 30 Kohlenstoffatomen, Triglyzeride, Wachse, Fettsäureester, natürliche Öle, Silikone.

Als eine Komponente des Fettkörpers enthalten die erfindungsgemäßen Zusammensetzungen bevorzugt mindestens einen linearen, gesättigten primären Alkohol mit 14 - 30 Kohlenstoffatomen.

Erfindungsgemäß bevorzugte lineare, gesättigte primäre Alkohole mit 14 - 30 Kohlenstoffatomen sind ausgewählt aus Myristylalkohol, Cetylalkohol, Stearylalkohol, 12-Hydroxystearylalkohol, Arachidylalkohol (Arachylalkohol), Behenylalkohol, Lignocerylalkohol, Cerylalkohol und Myricylalkohol und Mischungen hiervon, insbesondere technische Mischungen wie Arachidylalkohol und Behenylalkohol sowie Cetylalkohol und Stearylalkohol (Cetearylalkohol). Besonders bevorzugt sind Cetylalkohol, Stearylalkohol, Arachidylalkohol und Behenylalkohol und Mischungen hiervon. Besonders bevorzugt sind Mischungen aus Cetearylalkohol und Behenylalkohol.

Die vorgenannten Alkohole werden im Folgenden auch synonym als "Fettalkohole" bezeichnet.

Bevorzugte kosmetische Zusammensetzungen enthalten mindestens einen linearen, gesättigten primären Alkohol mit 14 - 30 Kohlenstoffatomen in einer Gesamtmenge von 0,5 bis 10,0 Gew.-%, bevorzugt 1,0 bis 8,0 Gew.-%, jeweils bezogen auf das Gesamtgewicht der Zusammensetzung.

Als weitere Fettstoffe können die erfindungsgemäßen Zusammensetzungen bevorzugt mindestens eine Fettsäure mit 6 bis 30 Kohlenstoffatomen enthalten.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol®871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge an Fettsäure beträgt dabei bevorzugt 0,1 bis 15 Gew.%, bezogen auf das gesamte Zusammensetzung. In einer besonders bevorzugten Ausführungsform beträgt die Menge 0,5 bis 10 Gew.%, wobei ganz besonders vorteilhaft Mengen von 1 bis 5 Gew.% sind.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Zu den natürlichen und synthetischen kosmetischen Ölkörpern, eignen sich bevorzugt:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle, insbesondere Capryl-/Caprinsäuretriglyzerid.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol®OE) können bevorzugt sein.
- Esteröle. Unter Esterölen sind zu verstehen die Ester von C₆ - C₃₀ - Fettsäuren mit C₂ - C₃₀-Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Triglyceriden gesättigter und gegebenenfalls hydroxylierter C₁₆₋₃₀-Fettsäuren, z. B. gehärteten Triglyceridfetten, insbesondere Triglyceride aus gehärtetem Palmöl.
- Phospholipiden, beispielsweise Sojalecithin, Ei-Lecithin und Kephalinen,
- Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/ oder Hydroxy-Gruppen enthalten, bevorzugt Substanzen mit den INCI-Bezeichnungen Dimethiconol, Amodimethicone oder Trimethylsilylamodimethicone.

Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf die gesamte erfindungsgemäße Zusammensetzung.

Weitere mögliche Inhaltsstoffe der erfindungsgemäßen kosmetischen Zusammensetzungen können über ihre Funktion definiert werden. Natürlich können manche Inhaltsstoffe auch multifunktionell sein. Bevorzugte Inhaltsstoffe der erfindungsgemäßen kosmetischen Zusammensetzungen können sein: Absorptionsmittel, antimikrobielle Stoffe, Bindemittel, Bleichmittel, Chelatbildner, Emollientien, Enthaarungsmittel und Wirkstoffe mit Haarwuchs inhibierender Wirkung, Feuchtigkeitsspender, Filmbildner, Farbstoffe, Konservierungsstoffe, Korrosionsschutzmittel, hautkühlende Wirkstoffe, pH-Wert-Regler/ Puffersubstanzen, Treibgase, Trübungsmittel, UV-Absorber/ Lichtfiltersubstanzen, Vergällungsmittel. Ferner können auch Kombinationen der vorgenannten Inhaltsstoffe in den erfindungsgemäß bevorzugten Zusammensetzungen enthalten sein.

Ein zweiter Gegenstand der Erfindung ist die Verwendung der Zusammensetzungen des ersten Erfindungsgegenstandes zur Pflege der Haut, insbesondere zur Pflege von Altershaut.

Ein dritter Gegenstand der Erfindung ist ein Verfahren zur Hautpflege, in dem eine Zusammensetzung des ersten Erfindungsgegenstandes auf die Haut aufgetragen und auf der Haut für einen Zeitraum von mindestens einer Stunde belassen wird.

### Beispiele

Es wurde folgende Emulsionen als Hautpflegemittel nach einem Standard Emulgierverfahren unter Einsatz der erfindungsgemäßen Inhaltsstoffe hergestellt:

| **Rohstoff** | **E1 [Gew.-%]** | **E2 [Gew.-%]** |
|---|---|---|
| 1,6-Hexandiol | 5,00 | 5,00 |
| Caprylic/Capric Triglyceride | 3,50 | - |
| Ethylhexyl Palmitate | - | 2,00 |
| Hexyllaurate | - | 2,00 |
| Glyceryl Stearate | 2,00 | 0,50 |
| Glycerin | 2,00 | 5,00 |
| Cetearyl Alcohol | 1,00 | 1,00 |
| Aluminum Starch Octenylsuccinate | 0,90 | 0,90 |
| 2-Phenoxyethanol | 0,90 | 0,90 |
| Sorbitol | 0,70 | 0,70 |
| Propylene Glycol | 0,60 | 0,60 |
| D,L-Panthenol | 0,50 | 0,50 |
| Dimethicone | 0,50 | - |
| Tocopheryl Acetate | 0,50 | 0,50 |
| Butylene Glycol | 0,50 | - |
| Sodium Acrylate/Sodium Acryloyldimethyl Taurate Copolymer | 0,38 | 0,19 |
| Sodium Carbomer | 0,35 | 0,34 |
| Potassium Cetyl Phosphate | 0,31 | 0,31 |
| Polyisobutene | 0,28 | 0,14 |
| Hydrogenated Palm Glycerides | 0,19 | 0,19 |
| D,L-alpha-Bisabolol | 0,09 | 0,09 |
| Sorbitan Oleate | 0,03 | 0,02 |
| Caprylyl/Capryl Glucoside | 0,02 | 0,01 |
| Camellia Sinensis Leaf Extract | 0,02 | 0,02 |
| 1,10-Decanediol | 0,01 | 0,01 |
| 10-Hydroxydecanoic Acid | 0,01 | 0,01 |
| Sebacic Acid | 0,01 | 0,01 |
| Pantolactone | 0,01 | 0,01 |
| Panax Ginseng Root Extract | 0,01 | 0,01 |
| Pinus Sylvestris Bark Extract | 0,01 | 0,01 |
| Ribes Nigrum (Black Currant) Leaf Extract | 0,01 | 0,01 |
| Parfum (Fragrance) | 0,20 | 0,20 |
| Wasser | ad 100 | ad 100 |

Die Zusammensetzungen wiesen eine gute Lagerstabilität auf. Sie ließen sich auf der Haut leicht verteilen und zeigten eine antibakterielle Wirkung.

## Patentansprüche

1. Kosmetische Zusammensetzung in Form einer Emulsion, enthaltend
a) mindestens ein Oligomeres einer Flavan-3-ol-Verbindung
und
b) mindestens eine Verbindung umfassend mindestens ein Strukturfragment der Formel (T1) 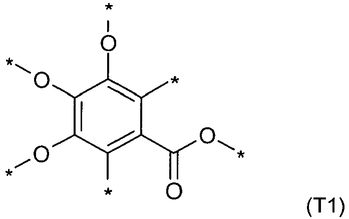 worin eine mit einem * gekennzeichnete Bindung für eine freie Valenz des Strukturfragments der Verbindung steht
und
c) Ginsengextrakt
und
d) mindestens ein Salz von C₁₂₋₂₀-Alkylphosphat, insbesondere ein Salz von Cetylphosphat,
und
e) mindestens ein C₁₄₋₂₀-Mono- oder Diacylglycerid, bevorzugt mindestens ein C₁₆₋₁₈-Mono- oder Diacylglycerid, besonders bevorzugt ausgewählt aus gehärteten Palmölglyceriden,
und
f) Wasser,
**dadurch gekennzeichnet, dass** die Salze von C₁₂₋₂₀-Alkylphosphat und die C₁₄₋₂₀-Mono- und C₁₄₋₂₀-Diacylglyceride in einem Gewichtsverhältnis C₁₂₋₂₀-Alkylphosphat-Salz zu besagtem Glycerid von 3 zu 1 bis 1 zu 1, enthalten sind.

2. Kosmetische Zusammensetzung gemäß Anspruch 1 **dadurch gekennzeichnet, dass** die Flavan-3-ol-Verbindungen aus mindestens einem oligomeren Proanthocyanidin ausgewählt wird.

3. Kosmetische Zusammensetzung gemäß Anspruch 1 oder 2, **dadurch gekennzeichnet, dass** es einen (insbesondere mit Wasser und/oder Ethanol als Extraktionsmittel gewonnenen) Extrakt der Blätter von *Ribes Nigrum,* enthaltend mindestens ein oligomeres Proanthocyanidin, enthält.

4. Kosmetische Zusammensetzung gemäß einem der Ansprüche 1 bis 3, **dadurch gekennzeichnet, dass** die Oligomere einer Flavan-3-ol-Verbindung bezogen auf die gesamte Zusammensetzung in einer Gesamtmenge von 0,00005 Gew.-% bis 0,1 Gew.-%, bevorzugt von 0,0001 Gew.-% bis 0,05 Gew.-%, enthalten sind.

5. Kosmetische Zusammensetzung gemäß einem der Ansprüche 1 bis 4, **dadurch gekennzeichnet, dass** ein (insbesondere mit Wasser und/oder Ethanol als Extraktionsmittel gewonnenen) Extrakt der Rinde von *Pinus Sylvestris,* enthaltend mindestens eine Verbindung der obigen Formel (T1), enthalten ist.

6. Kosmetische Zusammensetzung gemäß einem der Ansprüche 1 bis 5 **dadurch gekennzeichnet, dass** die Verbindungen der Formel (T1) bezogen auf die gesamte Zusammensetzung in einer Gesamtmenge von 0,00005 Gew.-% bis 0,1 Gew.-%, bevorzugt von 0,0001 Gew.-% bis 0,05 Gew.-%, enthalten sind.

7. Kosmetische Zusammensetzung gemäß einem der Ansprüche 1 bis 6 **dadurch gekennzeichnet, dass** der Ginsengextrakt mindestens ein Sapogenin enthält.

8. Kosmetische Zusammensetzung nach einem der Ansprüche 1 bis 7, **dadurch gekennzeichnet, dass** die Salze von C₁₂₋₂₀-Alkylphosphat, insbesondere die Salze von Cetylphosphat,, in einer Gesamtmenge von 0,2 bis 2,0 Gew.-%, bevorzugt 0,3 bis 1,8 Gew.-%, besonders bevorzugt 0,3 bis 0,7 Gew.-%, jeweils bezogen auf das Gesamtgewicht der Zusammensetzung, enthalten sind.

9. Kosmetische Zusammensetzung gemäß einem der Ansprüche 1 bis 8, **dadurch gekennzeichnet, dass** die C₁₄₋₂₀-Mono- oder Diacylglyceride, bevorzugt die C₁₆₋₁₈-Mono- oder Diacylglyceride, besonders bevorzugt die gehärteten Palmölglyceride, in einer Gesamtmenge von 0,1 bis 1,7 Gew.-% bezogen auf das Gesamtgewicht der Zusammensetzung, enthalten sind.

10. Kosmetische Zusammensetzung gemäß einem der Ansprüche 1 bis 9, **dadurch gekennzeichnet, dass** die Salze von C₁₂₋₂₀-Alkylphosphat (insbesondere die Salze von Cetylphosphat) und die C₁₄₋₂₀-Mono- und C₁₄₋₂₀-Diacylglyceride bevorzugt in einem Gewichtsverhältnis C₁₂₋₂₀-Alkylphosphat-Salz zu besagtem Glycerid von 2,5 zu 1 bis 1 zu 1, enthalten sind.

11. Kosmetische Zusammensetzung gemäß einem der Ansprüche 1 bis 10, **dadurch gekennzeichnet, dass** zusätzlich mindestens ein Verdickungsmittel enthält, ausgewählt aus mindestens einem Polymer, welches durch Polymerisation von zumindest 2-Methyl-2[(1-oxo-2-propenyl)amino]-1-propansulfonsäure (AMPS) erhalten wird.

## Claims

1. A cosmetic composition in the form of an emulsion, containing
a) at least one oligomer of a flavan-3-ol compound and
b) at least one compound comprising at least one structure fragment of formula (T1) 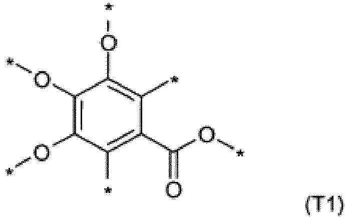 in which a compound bond indicated by a * represents a free valency of the structure fragment of the compound
and
c) ginseng extract and
d) at least one salt of C₁₂₋₂₀ alkyl phosphate, in particular a salt of cetyl phosphate, and
e) at least one C₁₄₋₂₀ monoglyceride or diglyceride, preferably at least one C₁₆₋₁₈ monoglyceride or diglyceride, particularly preferably selected from hydrogenated palm glycerides,
and
f) water,
**characterized in that** the salts of C₁₂₋₂₀ alkyl phosphate and the C₁₄₋₂₀ monoglycerides and C₁₄₋₂₀
diglycerides are contained in a weight ratio of C₁₂₋₂₀ alkyl phosphate salt to said glyceride of from 3:1 to 1:1.

2. The cosmetic composition according to claim 1, **characterized in that** the flavan-3-ol compounds are selected from at least one oligomeric proanthocyanidin.

3. The cosmetic composition according to claim 1 or 2, **characterized in that** it contains an extract (obtained in particular using water and/or ethanol as extraction means) of leaves of *Ribes nigrum* containing at least one oligomeric proanthocyanidin.

4. The cosmetic composition according to one of claims 1 to 3, **characterized in that** the oligomers of a flavan-3-ol compound are contained in a total amount of from 0.00005 to 0.1 wt.%, preferably from 0.0001 to 0.05 wt.%, based on the total composition.

5. The cosmetic composition according to one of claims 1 to 4, **characterized in that** it contains an extract (obtained in particular using water and/or ethanol as extraction means) of bark of *Pinus sylvestris* containing at least one compound of the above formula (T1).

6. The cosmetic composition according to one of claims 1 to 5, **characterized in that** the compounds of formula (T1) are contained in a total amount of from 0.00005 to 0.1 wt.%, preferably from 0.0001 to 0.05 wt.%, based on the total composition.

7. The cosmetic composition according to one of claims 1 to 6, **characterized in that** the ginseng extract contains at least one sapogenin.

8. The cosmetic composition according to one of claims 1 to 7, **characterized in that** the salts of C₁₂₋₂₀ alkyl phosphate, in particular the salts of cetyl phosphate, are contained in a total amount of from 0.2 to 2.0 wt.%, preferably from 0.3 to 1.8 wt.%, particularly preferably from 0.3 to 0.7 wt.%, in each case based on the total weight of the composition.

9. The cosmetic composition according to one of claims 1 to 8, **characterized in that** the C₁₄₋₂₀ monoglycerides or diglycerides, preferably the C₁₆₋₁₈ monoglycerides or diglycerides, particularly preferably the hydrogenated palm glycerides, are contained in a total amount of from 0.1 to 1.7 wt.%, based on the total weight of the composition.

10. The cosmetic composition according to one of claims 1 to 9, **characterized in that** the salts of C₁₂₋₂₀ alkyl phosphate (in particular the salts of cetyl phosphate) and the C₁₄₋₂₀ monoglycerides and C₁₄₋₂₀ diglycerides are preferably contained in a weight ratio of C₁₂₋₂₀ alkyl phosphate salt to said glyceride of from 2.5:1 to 1:1.

11. The cosmetic composition according to one of claims 1 to 10, **characterized in that** additionally at least one thickener is contained, selected from at least one polymer which is obtained by polymerization of at least 2-methyl-2[(1-oxo-2-propenyl)amino]-1 propane sulfonic acid (AMPS).

## Revendications

1. Composition cosmétique sous forme d'émulsion, contenant
a) au moins un oligomère d'un composé de flavan-3-ol
et
b) au moins un composé comprenant au moins un fragment structurel de formule (T1) 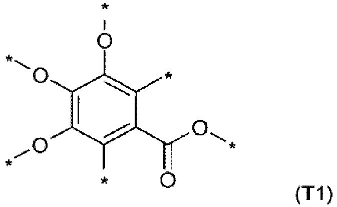 où une liaison marquée d'un * représente une valence libre du fragment structurel du composé
et
c) de l'extrait de ginseng
et
d) au moins un sel de phosphate d'alkyle en C₁₂₋₂₀, en particulier un sel de phosphate de cétyle,
et
e) au moins un mono- ou diglycéride d'acyle en C₁₄₋₂₀, de préférence au moins un mono- ou diglycéride d'acyle en C₁₆₋₁₈, plus préférentiellement choisi parmi les glycérides d'huile de palme durcis,
et
f) de l'eau,
**caractérisée en ce que** les sels de phosphate d'alkyle en C₁₂₋₂₀ et les monoglycérides d'acyle en C₁₄₋₂₀ et diglycérides d'acyle en C₁₄₋₂₀ sont présents dans un rapport en poids du sel de phosphate d'alkyle en C₁₂₋₂₀ audit glycéride situé dans la plage allant de 3:1, à 1:1.

2. Composition cosmétique selon la revendication 1, **caractérisée en ce que** les composés flavan-3-ol sont choisis parmi au moins une proanthocyanidine oligomère.

3. Composition cosmétique selon la revendication 1 ou 2, **caractérisée en ce qu'**elle contient un extrait de feuilles de *Ribes Nigrum* (obtenu en particulier avec de l'eau et/ou de l'éthanol comme agent d'extraction) contenant au moins une proanthocyanidine oligomérique.

4. Composition cosmétique selon l'une quelconque des revendications 1 à 3, **caractérisée en ce que** les oligomères d'un composé de flavan-3-ol sont présents en une quantité totale, rapportée à la composition totale, située dans la plage allant de 0,00005 % en poids à 0,1 % en poids, de préférence de 0,0001 % en poids à 0,05 % en poids.

5. Composition cosmétique selon l'une quelconque des revendications 1 à 4, **caractérisée en ce qu'**elle contient un extrait d'écorce de *Pinus Sylvestris* (obtenu en particulier avec de l'eau et/ou de l'éthanol comme agent d'extraction) contenant au moins un composé de formule (T1) ci-dessus.

6. Composition cosmétique selon l'une quelconque des revendications 1 à 5, **caractérisée en ce que** les composés de formule (T1) sont présents en une quantité totale, rapportée à la composition totale, située dans la plage allant de 0,00005 % en poids à 0,1 % en poids, de préférence de 0,0001 % en poids à 0,05 % en poids.

7. Composition cosmétique selon l'une quelconque des revendications 1 à 6, **caractérisée en ce que** l'extrait de ginseng contient au moins une sapogénine.

8. Composition cosmétique selon l'une quelconque des revendications 1 à 7, **caractérisée en ce que** les sels de phosphate d'alkyle en C₁₂₋₂₀, en particulier les sels de phosphate de cétyle, sont présents en une quantité totale située dans la plage allant de 0,2 à 2,0 % en poids, de préférence 0,3 à 1,8 % en poids, plus préférentiellement de 0,3 à 0,7 % en poids, rapportée au poids total de la composition.

9. Composition cosmétique selon l'une quelconque des revendications 1 à 8, **caractérisée en ce que** les mono- ou diglycérides d'acyle en C₁₄₋₂₀, de préférence les mono- ou diglycérides d'acyle en C₁₆₋₁₈, plus préférentiellement les glycérides d'huile de palme durcis, sont présents en une quantité totale située dans la plage allant de 0,1 à 1,7 % en poids, rapportée au poids total de la composition.

10. Composition cosmétique selon l'une quelconque des revendications 1 à 9, **caractérisée en ce que** les sels de phosphate d'alkyle en C₁₂₋₂₀ (en particulier les sels de phosphate de cétyle) et les mono- et diglycérides d'acyle en C₁₄₋₂₀, sont de préférence présents dans un rapport en poids du sel de phosphate d'alkyle en C₁₂₋₂₀ audit glycéride situé dans la plage allant de 2,5:1 à 1:1.

11. Composition cosmétique selon l'une quelconque des revendications 1 à 10, **caractérisée en ce qu'**elle contient en outre au moins un agent épaississant, choisi parmi au moins un polymère obtenu par polymérisation d'au moins l'acide 2-méthyl-2[(1-oxo-2-propényl)amino]-1-propanesulfonique (AMPS).
